# FASCICULE DE BREVET EUROPEEN

(11) **EP 0 434 522 B1**
(45) Date de publication et mention de la délivrance du brevet: **01.03.1995**
(21) Numéro de dépôt: 90403597.9
(22) Date de dépôt: 14.12.1990
(51) Int. Cl.: A61L 9/01

(54) **Utilisation d'un désodorisant à base d'ester d'acide undécylénique pour la désodorisation des boues des stations d'épuration.**
Verwendung von Undecylensäureester enthaltendes Desodorierungsmittel für Kläranlagenschlamm.
Use of a deodorizer based on undecylenic acid ester for sludge in water waste plants.

(30) Priorité: 19.12.1989 FR 8916792
(43) Date de publication de la demande: 26.06.1991
(73) Titulaire: DELTA AGRO INDUSTRIES, F-75015 Paris (FR); ELF ATOCHEM S.A., 92800 Puteaux (FR)
(72) Inventeur: Menassa, Aimé, Paris 16ème (FR); Caupin, Henri, F-78000 Versailles (FR)
(74) Mandataire: Rochet, Michel

(56) Documents cités:
- DE-A- 2 263 509
- US-A- 4 517 919

## Description

L'invention a pour objet l'utilisation d'un désodorisant pour des boues des stations d'épuration.

Dans le domaine de la désodorisation, on sait qu'il est possible d'atténuer certaines odeurs indésirables par l'emploi de compositions à base d'ester d'acide undécylénique.

Ainsi, dans DE-A-2 263 509, on propose de prévenir la formation des microorganismes responsables des odeurs corporelles chez l'Homme en utilisant une composition déodorante complexe comprenant un ester alkylique (C₁-C₄) d'acide undécylénique.

Les stations d'épuration sont sources de polluants odorants, dont les origines se trouvent dans les effluents aussi bien en amont des traitements qu'en aval.

En amont de la station, l'égout est le lieu privilégié des fermentations influencées par la température des effluents, la vitesse de l'eau, le type de réseau ...

Les dégagements d'odeurs sont moins bien localisés au sein de la station.

La réduction des risques d'émission constitue les premières dispositions de lutte contre les odeurs et passe par :
- l'élimination des polluants volatils contenus dans l'effluent ;
- la limitation des phénomènes de formation des composés chimiques odorants ;
- la limitation des phénomènes physiques de passage des phases aqueuses à gazeuses.

Cette lutte contre les odeurs se traduit généralement par l'utilisation, en amont de la station d'épuration, de l'une ou l'autre des techniques suivantes :
- injection de peroxyde d'hydrogène ;
- injection d'oxygène pur ;
- injection de sel de fer (sulfate ferreux complexant les sulfures sous forme de sulfure de fer).

En fait les principales sources d'odeurs non maîtrisées dans les stations d'épuration sont liées à la formation de boues.

L'invention propose donc l'utilisation d'une substance désodorisante pour la désodorisation de boues d'épuration, caractérisée en ce que ladite substance comprend un ester alkylique ou polyoxyalkylénique d'acide undécylénique renfermant de 2 à 10 motifs oxyalkylène.

Parmi ces produits, l'invention concerne tout particulièrement l'utilisation des esters alkyliques en C₁ et C₁₂ et les esters de polyoxyéthylène, polyoxypropylène et poly(oxyéthylène) (oxypropylène) dudit acide undécylénique.

La substance absorbante utilisée conformément a l'invention peut être constituée d'un seul des produits précités ou d'un mélange desdits produits, ce ou ces produits pouvant euxmêmes être mis en oeuvre tels quels ou sous forme de solution ou suspension, ou encore sous forme adsorbée sur un support tel que par exemple des particules d'argile.

D'une manière générale, les esters undécyléniques sont efficaces à très faible dose, par exemple de l'ordre de 0,01 à 5 % par rapport à la masse à traiter.

Les courbes annexées et qui constituent des exemples d'illustration non limitatifs, montrent l'efficacité de certains esters alkyléniques et polyoxyéthyléniques d'acide undécylénique.

Dans ces courbes, l'axe des abscisses représente le taux d'incorporation de l'ester undécylénique, l'axe des ordonnées représentant le degré de perception olfactive de l'odeur, les valeurs 1 à 6 signifiant respectivement : néant, très faible, faible, moyen, fort et très fort.

Sur ces figures, la courbe supérieure correspond à l'odeur de la boue elle-même, la courbe inférieure, éventuellement présente, correspondant à l'odeur de l'ester.

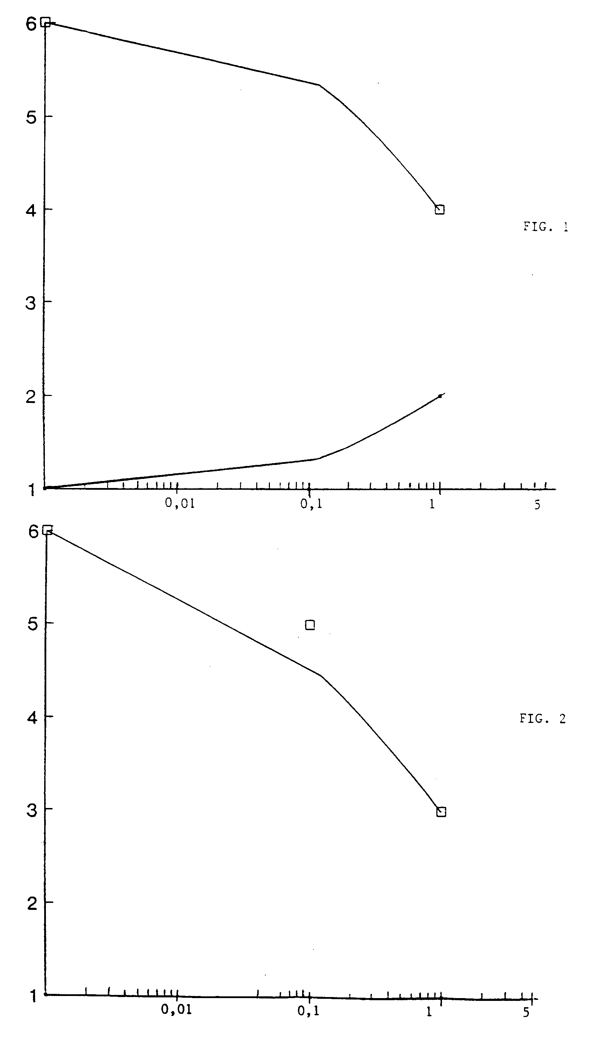

Les courbes des figures 1 à 3, conformes à l'invention, montrent que la réduction de la perception de l'odeur de la boue n'est pas remplacée de manière sensible par la perception d'une odeur liée à l'ester lui-même.

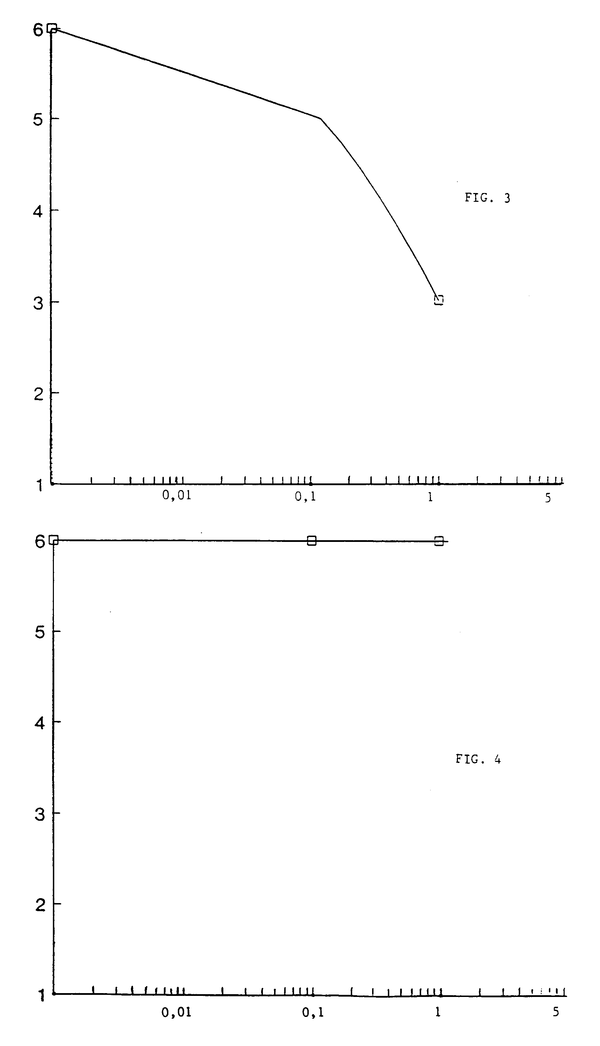

A titre de comparaison, la figure 4 montre qu'avec un ester polyoxyéthylénique de l'acide undécylénique renfermant 12 motifs d'oxyde d'éthylène, aucune réduction de la perception de l'odeur de la boue ne peut être constatée.

Les courbes 1 à 3 correspondent respectivement à l'undécylénate de méthyle, à l'ester polyoxyéthylénique d'acide undécylénique à 8 motifs oxyéthylène et à l'ester du même acide à 10 motifs oxyéthylène.

## Revendications

1. Utilisation d'un désodorisant comprenant un ester alkylique ou polyoxyalkylénique renfermant 2 à 10 motifs oxyalkylène d'acide undécylénique pour la désodorisation des boues de stations d'épuration.

2. Utilisation selon la revendication 1 caractérisée en ce que le désodorisant est choisi parmi les esters alkyliques en C₁ - C₁₂ et les esters de polyoxyéthylène, polyoxypropylène et poly(oxyéthylène)(oxypropylène) de l'acide undécylénique.

3. Boues de stations d'épuration, renfermant de 0,01 à 5 % en poids d'un désodorisant selon l'une quelconque des revendications 1 ou 2.

## Claims

1. Use of a deodorant comprising an alkyl ester or a polyoxalkylene ester containing 2 to 10 oxyalkylene units of undecylenic acid for deodorizing the sludges from purification stations.

2. Use according to Claim 1, characterized in that the deodorant is chosen from the C₁-C₁₂ alkyl esters and the polyoxyethylene, polyoxypropylene and poly(oxyethylene) (oxypropylene) esters of undecylenic acid.

3. Sludges from purification stations, containing from 0.01 to 5 % by weight of a deodorant according to either of Claims 1 and 2.

## Patentansprüche

1. Verwendung eines Desodorierungsmittels, das einen Alkyl- oder Polyoxyalkylenester mit 2 bis 10 Oxyalkyleneinheiten der Undecylensäure enthält, zur Desodorierung von Kläranlagenschlamm.

2. Verwendung nach Anspruch 1, dadurch gekennzeichnet, daß das Desodorierungsmittel ausgewählt wird aus C₁-C₁₂-Alkylestern und Polyoxyethylen-, Polyoxypropylen- und Poly(oxyethylen)(oxypropylen)estern von Undecylensäure.

3. Kläranlagenschlamm, enthaltend 0,01 bis 5 Gew.-% eines Desodorierungsmittels nach Anspruch 1 oder 2.
